# EUROPEAN PATENT APPLICATION

(11) **EP 3 650 050 A1**
(43) Date of publication of application: **13.05.2020**
(21) Application number: 19206308.9
(22) Date of filing: 30.10.2019
(51) Int. Cl.: A61L 2/07, A61L 2/24, A61L 2/28

(54) **MODULAR CYCLE STERILIZATION PROCESS**

(30) Priority: 08.11.2018 DE 102018127997
(71) Applicant: MMM Münchener Medizin Mechanik GmbH, 82152 Planegg (DE)
(72) Inventor: Eibl, Robert, 92539 Schönsee (DE); Göllner, Gerald, 92539 Schönsee (DE); Bönisch, Martin, 80687 München (DE); Vogt, Alexander, 86163 Augsburg (DE)
(74) Representative: Wittmann, Ernst-Ulrich

(57) **Abstract**

Method of providing a customizable sterilization process for a sterilizer load or device comprising the steps of (a) specifying the device or sterilizer load by its characterizing parameters from a list of device parameters, wherein each device parameter possesses an identification code/tag; (b) providing a list for different process steps including air removal, sterilization and drying, together with individual process settings for each individual process step; (c) using the identification code/tag for selecting a sterilization sequence including the process steps and the individual process settings from the list; (d) executing the sterilization sequence.

## Description

Reusable medical equipment, such as surgical instruments, implants, medical devices, as well as any other equipment used in food handling facilities, clean rooms, sterile manufacturing rooms, beauty salons and tattoo parlours, wherever contamination causes concern, undergoes sterilization procedures, wherein sterilization is intended as the removal of bacteria or living organisms or contaminants which are hazardous to health or generically unwanted from any of such devices.

A strong perspective on the subject of developing sterilization systems, methods or procedures is given by those fields where maintaining affordable and effective infrastructures to offer health care to the population is a main focus. In this respect, healthcare is under enormous cost pressure all over the world. Operating health facilities cost-effectively and at a high safety level also influences key processes and puts pressure on the Central Sterile Services Department (CSSD) or the Reprocessing Units for Medical Devices (RUMED). Apparatuses for instrument reprocessing have to withstand the legal high requirements and work optimally in terms of time and resources consumption.

Most of the instruments used in surgery, medical care facilities, food handling facilities, manufacturing facilities, etc..., are u.a. treated in a sterilizer.

A modern sterilization cycle e.g. for medical devices usually consists of several steps, such as "deaeration phase", "heating", "holding phase" and "drying". In addition to the holding phase, the "deaeration or air removal phase" is of particular importance. Air as a non-condensable gas impedes heat transfer or the access of steam to complex, highly structured recesses or cavities (U. Kaiser, 2005). This effect is particularly serious, especially in the case of porous goods (for example fabric, linens) but also in the case of goods with lumens or hollow bodies.

Modern medical devices or food handling or semiconductor manufacturing tools or other similar instrumentation often have cavities that are for their structural complexity difficult to vent. Accordingly, for example, the venting phase of a sterilization process must be accurately designed for each different device (e.g., multiple aspiration of the sterilization chamber by means of evacuation and subsequent steam inflow, low pressure reversal points) to deliver effective results, becoming considerably expensive. This in turn results in both higher media consumption and longer cycle times than existing standard cycles.

As early as 2006, Kaiser and H. Vogel proposed a hollow-body penetration factor (HPF), which allows a quantitative classification of the venting of hollow bodies in steam sterilization processes.

According to ISO 17665-3 (2013), a so-called vapor penetration resistance is assigned to different product families in order to classify the products in terms of deaeration and vapor penetration behavior.

According to the prior art, the test for the suitability of a sterilization cycle for a particular item to be sterilized is carried out by a process validation (for example by means of thermometric measurement) and a test with hollow body loading (DIN EN 285, 2016). The normatively required test with hollow body loading with a standardized "pattern" hollow body, however, is not demanding enough for many medical devices or food handling or semiconductor manufacturing tools or other similar instrumentation. In practice, the selection of a suitable hollow body system and appropriate sterilization process is the responsibility of an, often, untrained operator and is therefore not repeatable and with high likelihood of ineffectiveness, introducing potential health hazards or risks of defective operations of manufactured devices owing to contamination.

Often, therefore, in the attempt to limit risks connected to the inexperience of operators a worst-case cycle is run, a choice which does not always represent the most economical or effective option in terms of time and resources consumption.

In routine operation, a daily steam penetration test (B & D test) is run. The cycle of the B & D test must be consistent with the productive sterilization cycles with respect to the venting phase. The routine controls defined by the operator for each cycle during the process validation by means of commercial BMS (Batch Monitoring System) are typically not adapted to the respective sterilization cycles and individual medical devices or food handling or semiconductor manufacturing tools or other similar instrumentation. If a BMS with insufficient venting resistance is selected, if no errors are detected, or if the venting resistance is too high, problems with batch release are likely to result wherein insufficient color change of the indicator of a BMS is achieved.

Another important factor is the final drying step. The sterilized material should be so dry after the sterilization process that recontamination can be largely ruled out. The drying is achieved according to the prior art on the pressure-dependent sending of the boiling point by means of vacuum drying. Depending on the type of loading (as defined by categories of parameters specifying a device, such as e.g. design, material, weight, packaging) different drying times and process management may be necessary. Commonly used are both simple drying processes with evacuation and holding phase as well as more complex drying processes with multiple intermediate vents and evacuations to allow replacement of the moist air. This results in both higher media consumption and longer cycle times.

The present invention overcomes at least partially the disadvantages of the prior art by introducing a method of providing a customizable sterilization process for a sterilizer load or device which can be customized and adapted to store data for further use.

According to the present invention, the method refers to a process comprising the steps of (a) specifying the device or sterilizer load by its characterizing parameters from a list of device parameters, wherein each device parameter possesses an identification code/tag; (b) providing a list for different process steps including air removal, sterilization and drying, together with individual process settings for each individual process step; (c) using the identification code/tag for selecting a sterilization sequence including the process steps and the individual process settings from the list; (d) executing the sterilization sequence.

According to the present invention, the terminology "sterilizer load" or "device" is used in the entirety of this document, sometimes also interchangeably, to refer to any load which undergoes the sterilization process, or which is intended for the sterilization process.

A sterilization sequence, or process, or sometimes cycle, is a series of actions or operations needed to achieve a set of specific requirements for sterility, usually defined, listed or outlined in a standard, and in particular, for example, the harmonized European standard EN 556-1 specifying requirements for designating a terminally-sterilized device as sterile, also adopted in other countries such as Australia and China, or the ISO/TS 19930:2017, or the DIN EN 285:2016-05 or any of a portfolio of standards and harmonized European standards for development, validation and control of sterilization systems, processes, equipment and personnel.

A sterilizer is an apparatus designed to carry on processes and methods for sterilization, such as for example the method according to the present invention. Apparatuses for sterilizations may vary within a broad range according to the specific application and specific industrial requirements.

According to the present invention, a device parameter is a feature of the device which specifies and therefore serves to identify a sterilizer load or device by providing attributes which describe the load or device according to a number of categories; according to the load or device, a number of predefined categories is given or chosen, wherein then subsequently an attribute, or a further specifier of a specific feature of a load or device within each category, is chosen in order to accurately describe the load or device.

The specification of a number of device parameters or attributes, accordingly distributed within each category and carefully selected, has the advantage of making the subsequent sterilization steps customized to the specifications of the load or device, and therefore conferring a higher accuracy and safety of the sterilization process, effectively reducing times and costs expenditure by appropriately assigning a sterilization sequence. The method as disclosed according to the present invention should give the operators the freedom to configure the sterilization process to match the treated medical devices. This is important because different devices pose different challenges for the sterilization sequence.

In particular, according to the present invention, the list of device parameters comprises at least one of device parameters chosen from a list of categories of device parameters, wherein the categories range e.g. from A to Z.

The number of categories for a device or for a group or class of devices can be configured as standard or vary to only highlight the categories which are relevant to a particular device. The accurate choice of categories and the choice of parameters allows for an accurate description of the device, which leads to a better choice of a sterilization sequence.

According to the present invention each category of device parameters comprises e.g. from 1 to n device parameters, wherein the number of device parameters is defined by the number of attributes of a given category.

The number of device parameters can be given, defined according to the field of application of the sterilization process, and/or continuously or periodically updated to fit the latest technological advancements. Such device parameter as defined within a category correspond to the attributes of a device or a sterilizer load, therefore such number may vary according to the device or sterilizer load and to the specific field of application of the sterilization process.

This continuous customization, together with the large number of possibilities for providing a custom sterilization sequence successful for the specific device for which it is generated, shows the flexibility of the method according to the present invention. Updating the attributes within each category ensures a great adaptability to every load, with a reduced risk of operator-dependent mistakes.

According to one embodiment of the invention, the identification code/tag corresponds to the combination of a category identifier e.g. from A to Z and the number of device parameters per category e. g. from 1 to *n.*

Each device parameter according to the present invention has therefore a recognizable identifier, so that a number of device parameters clearly identifies a device or sterilizer load with great accuracy.

In particular, according to the present invention the list of categories of device parameters includes at least one of e.g. design, including structural parameters such as e.g. the presence of cavities and/or recesses and/or protrusions; weight; size; material or materials of the device; wrapping of the device; container of the device; sterilization temperature and sterilization time of the device.

Within the understanding of the present invention, there is a strong correlation between the characterizing attributes of a device to be sterilized, grouped into a number of categories, as explained above, and the process steps required to determine and executing an appropriate sterilization sequence.

Said process steps consist of at least one of air removal, sterilization and drying; an air removal step is intended as such step by which air is removed from the device, its cavities and recesses and any inner structure, according to methods of air removal such as for example vacuum air removal, in order to facilitate or render possible any of the subsequent steps, such as, for example the sterilization step, requiring steam penetration and therefore air-free environment.

In particular during the sterilization step as intended in the present invention, steam may be used in order to expose every part or portion of a device to a suitable biocidal temperature. As a consequence, a step of drying may be required, wherein any residual moisture has to be removed to rule out risks of recontamination.

As the efficiency of these steps is connected with the choosing of such steps appropriately to meet the requirements of each device attributes, according to the invention, the specifications of each device influence the choice of any sterilization step, determining the assembly of an appropriate sterilization sequence.

In the meaning of the present invention therefore, the general categories of devices as mentioned above cause varying requirements for the different steps of a sterilization cycle (see ISO/TS 17665-3 for comparison); the category design of a device, for example in the medical field, may vary from simple (like a bow, scalpel, etc.) to highly complex (drills, bored handles, robotic surgical instruments, etc.). Complex devices may incorporate long hollow channels, tortuous paths, etc. In addition, there are devices with porous properties like linen or filters.

The design has a major influence on the difficulty of air removal and steam penetration on all surfaces. In addition, the removal of condensate is also influenced. Therefore, the drying step is influenced by the design of the medical device as well.

The weight of medical devices does also vary very much. In addition, the combined weight of different instruments incorporated in a set on a tray or in a sterilization container may also vary. Finally, the amount of load in the sterilization chamber e.g. the number of sets sterilized at once changes from cycle to cycle.

The individual weight differences impact the air removal stage as well as the drying stage because of the condensate which forms during the process. For the same reason the differences in set weights and amount of load in the chamber influence mainly the drying stage, but they do also influence the air removal stage.

The main distinction in materials used for example in the field of medical devices is the one between metallic and non-metallic materials. These different materials have different thermal properties (most important the thermal conductivity). This has an influence on the air removal stage as well as the drying stage. In addition, the material might limit the sterilization temperature so that a 121°C-cycle will be used rather than a cycle with 134°C.

To prevent recontamination after the sterilization, the devices are contained in some form of sterile barrier system. These systems vary from simple wrapping paper (eg. crepe made of medical paper, wrapping made of synthetic fibres, double-layered wrapping, ...) to sealed pouches to reusable container systems. These different systems pose different requirements to the air removal stage and to the drying stage.

According to the present invention, and as briefly mentioned above, the list of attributes of device parameters for each category includes at least one from a list of specific attributes of a category, wherein e.g. the category design includes at least one of the attributes such as easy, medium, complex.

This contributes to the accuracy in the selection of the appropriate sterilization sequence.

As mentioned above, according to the present invention each device parameter has an associated influence on a given process step, wherein each device parameter is associated with a suitable process parameter for any given sterilization step, and wherein a sterilization sequence is therefore built by cross-correlating the device parameters with the corresponding sterilization steps.

This cross-correlation established between the device parameters and the process steps of the sterilization sequence through their influence on their accurate determination reveals the accuracy of the method and its aptness to customization.

The method according to the present invention comprises a specification step which in turn comprises at least one of the steps of (i) selecting a category from a list of categories of device parameters; (ii) selecting an attribute for the device parameter in the given category; (iii) repeating (i) and (ii) for each relevant category.

The specification step is the first step towards the making of a customized sterilization sequence. In the meaning of the present invention, specification is intended as the best approximation in describing a device or sterilizer load in terms of features belonging to a group of well-defined categories, and further specifying attributes within each category that better define said device or sterilizer load. The selection of a first category, for example A, comes first; then within said category an attribute from 1 to n is selected to refine the choice of category, as explained above.

According to the present invention, the method includes a further step of providing an automatic selection of a batch monitoring and indicator system suitable for a customized sterilization sequence, wherein the suitable batch monitoring and indicator system is identified by a code/tag.

To verify the accuracy/effectiveness of the method according to the present invention, several verification methods options are available, essentially grouped into three main categories: verification to be executed during an active sterilization process, verification to be executed after termination of an active sterilization sequence and verification on demand, together with any combination thereof. Any of these processes is conducted in strict adherence to a standard for validation of sterilization processes, for example the standard for validation of steam sterilization processes EN ISO 17665-1 or updated or similar ones with validity in different countries and/or in the specific application target industry.

Essentially a verification step provides a confirmation that the requirements for a specific sterilization process as intended in the standard or protocol of reference have been met through an objective and quantifiable evidence.

In some embodiments, the method according to the present invention includes a further step of monitoring and/or verification of the results of the executed sterilization sequence, wherein the monitoring and/or verification step is continuous, batch or on demand.

In an embodiment of the present invention the verification step is carried out by placing a commercial analogue indicator e.g. based on colour and/or turbidity change, such as an indicator paper, indicator test strips, indicator tape, indicator solution, spore ampoules, chemical indicator, steam chemical process indicator, biological indicator, self-contained biological indicator, Bowie-Dick test packs, or other indicator devices such as e.g. digital monitors/indicators.

An indicator in the meaning of the present invention provides indication on whether the specific conditions to kill a number of microorganisms, for example bacteria or fungi, as specified in a standard or regulations protocol followed in the specific country of application and according to the specific application, have been met in the sterilization process, within a level of confidence determined as safe within said standard or protocol.

Moist heat is known to terminate microorganisms by the mechanisms of enzymatic and structural protein denaturation, determining irreversible coagulation of said structures: in particular it is known in the art that the presence of moisture influences the temperature at which coagulation takes place and therefore the termination of the microorganisms by failure of their life supporting structures.

Nevertheless, dry heat sterilization is also effective for other type of contaminants and microorganisms for the termination of which moisture plays little or no relevant role.

The use of indicators therefore verifies the results of a sterilization sequence and guarantees the safety of the selected procedure, according to regulations and inherent needs of the specific application.

Moreover, said indicators are suited to be used at different steps of a sterilization sequence or used during the overall sequence, to detect overall failure or even specific failure of a specific step, isolated from the others, and preferably before or after or while the steriliser load is loaded in the sterilizer's sterilization environment. The use of indicators in the mentioned ways contributes to the maintenance of a sterilizer apparatus in efficient conditions, for example not only to monitor the sterilization effectiveness, but also to check for equipment leaks, so that results are achieved in compliance with the regulations and standards of reference and energy efficient solutions are adopted to prevent malfunction of the equipment and safety hazards to the end users of the sterilizer load or device after sterilization.

In one embodiment of the present invention, the verification of sterilization is carried out in parallel to a sterilization sequence.

This is usually carried out with an especially designed device to reproduce features of a sterilizer load or device, which undergoes the same sterilization sequence as the sterilizer load or device, and wherein the effectiveness of any process step can be monitored, checked, recorded and used for assessment of the safety and effectiveness of the main sterilization sequence.

In the meaning of the present invention, any of the steps of the sterilization sequence of air removal, sterilization, and drying may be performed more than once, each step also with different parameters.

In order to provide the most accurate sterilization results, a sterilization sequence is built that matches the needs of sterilization of each device or group of devices. This goal is reached by ensuring that each process step is carried out with a precise final result, which can be reached also by carrying out a basic step more than once in a sequence, or even after other basic steps have been performed or in a new cycle, with or without variation of process parameters from one repetition to the other.

This means that in a particular embodiment of the present invention, any step of the sterilization sequence has one or more alternative forms, wherein an alternative form includes a set of parameters, such as, e.g., method, pressure, duration, temperature, gradients, pulses.

This flexibility in the making of an as complex as necessary, as well as of an as simple as necessary, sterilization sequence allows the achievements of sterilization results that meet the criteria imposed by a specific protocol or standard for sterilization.

In particular, according to the present invention, the step of air removal is carried out according to one or more of suitable air removal methods e.g. pre-vacuum air removal, fractionated pre-vacuum air removal, gravitation method.

In the meaning of the present invention, and according to the definition of air removal step as given above, several suitable methods to achieve said air removal are available. According to the pre-vacuum and/or fractionated air removal method, air is mechanically removed through a series of vacuum and pressure pulses, allowing the steam to reach recesses and pores that would not be otherwise reachable by simple displacement. The gravitation method relies instead on air displacement through steam by means of gravity and through a drain port, therefore without mechanical assistance. The steam rises to the top of the environment within the sterilizer and gradually as it fills the room pushes the air out, reaching the sterilizer load. Each of these choices, as well as the choices of further parameters for the specific process step, are influenced by the specified device parameters, so that air removal methods suited to for example porous materials can be chosen for linen or fabric or filters and so on.

According to the method of the present invention, the step of air removal is carried out at a vacuum pressure in the range from 10 mbarto 200 mbar, and in particular from 20 mbarto 150 mbar, and preferably from 30 mbar to 100 mbar.

Said pressures or pressure ranges apply to different materials and design of a specific sterilizer load, with the advantage of customizing the air removal step and facilitating the subsequent application of steam and further sterilization procedures, and correspond to absolute values of pulse pressures. Clearly the air removal effect of the vacuum or pre-vacuum pulses is associated with the choice allowed through the method of the present invention, so that the required efficiency, given a specified device or sterilizer load, prompts the corresponding accurate choice of the most appropriate step parameter.

According to the present invention the step of air removal is carried out using steam pulses, such as e.g. atmospheric pulses, trans-atmospheric pulses, overpressure pulses.

Pulses are intended in the meaning of the present invention as alternating steam pressure injections and vacuum draws. Vacuum is produced to force steam in recesses where it would not be able to penetrate at different pressure, ensuring therefore that surfaces within tubes, cavities, pores are conveniently sterilized. Because the need for each sterilizer load is different depending on its design and other attributes, as mentioned above, several types of pulses are used, so that the process results in high efficiency and accordingly efficient energy consumption, without excessive use of resources when not necessary.

In the meaning of the present invention the step of air removal is carried out according to rising and/or falling gradients.

According to the influence of the device parameters, air removal is carried out using different gradients, so that the final cycle meets the requirements of sterilization in accordance with the specific requirements of the particular sterilizer load or device.

According to the present invention, the step of sterilization is carried out at temperatures in the range from 100°C to 160°C, alternatively from 110°C to 140°C, in particular from 120°C to 135°C and preferably at 121° C or 134° C.

Temperature ranges and preferred temperature values are necessary according to the different materials and sterilization requirements, therefore according to the standard protocols of reference. Several temperature cycles may be required to achieve the desired results according to the thermoplastic resistance of different materials.

Ideally saturated steam is better suited for achieving high temperatures and reducing sterilization times when according to the resistance of a given device material this is needed. The maintenance of the correct temperature for the sterilization requirement for an appropriate duration of time ensures that those microorganisms sensitive at said temperature are killed by the sterilization procedure. A selection of the correct combination of steam temperatures and durations of the steam pulses is therefore prompted by the specification of device parameters and attributes, to achieve accurate customization.

According to the method of the present invention, the step of sterilization has durations or holding times in the range from 1 min to 30 min, and alternatively from 2 min to 25 min, in particular from 3.5 min to 20 min, and preferably from 5 min to 10 min, according to the sterilization temperature.

This ensures a flexibility for the sterilization to the required standard of any given device or group of devices.

According to the method as disclosed in the present invention, the step of drying is carried out according to one or more of suitable drying methods e.g. vacuum drying, fractionated drying with air admission, fractionated drying with steam admission.

Drying, as mentioned above, is used to rule out residual water borne contamination within the confidence of the corresponding standard for a given sterilizer load.

Acoording to the invention, the step of drying is carried out at a vacuum pressure in the range from 10 mbar to 200 mbar, and in particular from 20 mbar to 150 mbar, and preferably from 30 mbarto 100 mbar.

In particular, said step of drying has durations in the range from 1 min to 60 min, and in particular from 5 min to 45 min, and preferably from 10 min to 30 min.

According to the present invention, the step of drying is carried out using vacuum drying in combination with air or steam pulses, such as e.g. atmospheric pulses, trans-atmospheric pulses, overpressure pulses, air pulses, steam pulses, wherein the number of pulses is in the range from above 0 to 10, and in particular from 2 to 8, and preferably from 4 to 6, and the duration of said pulses is in the range from above 0 min to 10 min, and in particularfrom 1 min to 8 min, and preferably from 2 min to 4 min and from above 4 min to 6 min, and wherein the air temperature is in the range from 5°C to 160°C, and in particular from 10°C to 140°C, and preferably from 20°C to 120°C, in particular in combination with improved convection, e.g. by ventilation.

The use of the method according to any of the preceding claims for the sterilization of devices in any private and public industries where a standard hygiene requirement has to be met, such as e.g. hospitals, clinics, dentistry and orthodontics laboratories, the semiconductor manufacturing industry, the food handling, producing and manufacturing industry, the space technology manufacturing industry, the pharmaceutical industry, clean room environments, life science laboratories, animal facilities, beauty and cosmetic treatments salon, tattoo parlours.

The advantage of a highly customizable method to provide a sterilization process, according to the present invention, results clearly in the broad possibility for application in a variety of fields, to meet a variety of standards and regulations.

By means of example only, representative embodiments of the method according to the present invention are explicated in the following examples and illustrated in the following figures.

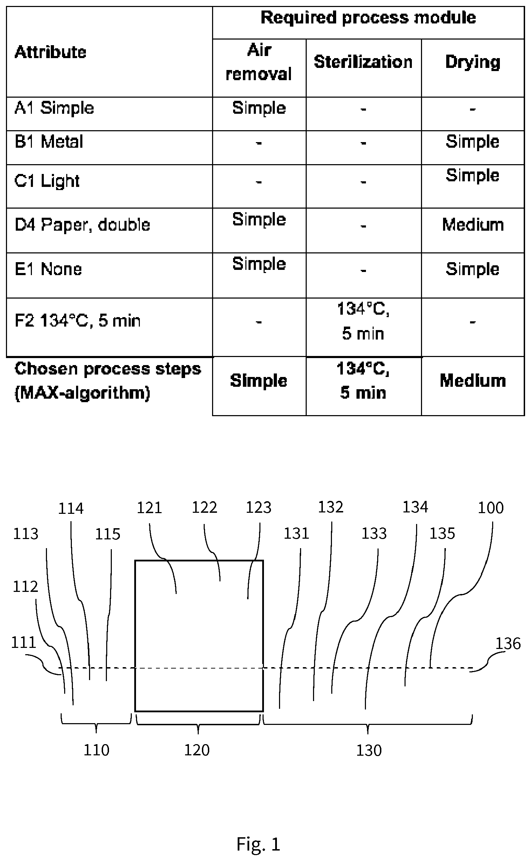

In the Figures and Examples, device parameters shown as attributes representing the device feature within each given category are listed, with the representation as explained above of letter corresponding to the category of the parameter and number reflecting the attribute of the device within the given category. The device parameters prompt the selection of a customized sterilization sequence by taking into account the influence that each of said device parameters has on the choice of a specific alternative form of a process step. Moreover, sterilization sequences and/or single sterilization process steps can be represented in graphs illustrating the changes in pressure and temperature overtime, as shown in the following figures. The frequency and duration intervals will vary depending on the chosen or assigned sterilization sequence and general sterilizer configuration, determined by parameters specification and algorithms calculation of the influence of the given parameters on the selection of a suitable alternative form of the process steps. In the Examples given below, and as mentioned in Figures 1 to 3, the evaluation of influences of the device parameters on the process steps is determined by an algorithm, and in the particular embodiments shown, by the MAX-algorithm.
- Figure 1:: Determination for the sterilization process sequence (table) and example of the resulting customized sequence (diagram) for a partial load with simple instruments, as explained in Example 1.
- Figure 2:: Determination for the sterilization process sequence (table) and example of the resulting customized sequence (diagram) for a full load with medium complex instruments, as explained in Example 2.
- Figure 3:: Determination for the sterilization process sequence (table) and example of the resulting customized sequence (diagram) for a full load with heavy and complex instruments, as explained in Example 3.
- Figure 4:: Table (a) shows categories (A to Z) and attributes (1 to n) specifying a device or sterilizer load; table (b) shows the influence that said device specifications or parameters have on the selection of a specific process step or alternative forms thereof, as calculated by an algorithm.
- Figure 5:: Overview of several alternative forms that the distinct process steps of air removal, sterilization and drying can take, which can be utilized according to the specification of device parameters as shown, respectively, in Figures and Examples 1 to 3.
- Figure 6:: Flow chart depicting a typical process leading to the customized assembly of an *ad hoc* sterilization sequence, together with execution and verification steps, according to the present invention, devised as to be executed even by an often inexperienced or untrained operator.

### Example 1: Partial load with simple instruments as shown in Figure 1

The sterilizer is loaded partially (respect to full capacity) with sets of simple instruments mostly made of metal (e.g. speculum, forceps, scissors, spatula, ...). Upon specification of the sterilizer load, the challenge for air removal is detected as low, so the simple air removal module is assigned.

The instruments are put on a polycarbonate tray and double wrapped with crepe paper. The challenge for drying is detected as medium, so medium drying is assigned.

According to these attributes, the goods should be sterilized at 134° for 5 minutes.

The determination for the sterilization process sequence (table) and the customized sequence (graph) would look as shown in Figure 1.

In particular, Figure 1 is concerned with obtaining a customized sterilization sequence for a partial load with simple instruments. The table in Figure 1 reports the device parameters as specified, deriving from a selection of categories and attributes within each category; the graph in Figure 1 shows a graphical representation of the sterilization sequence, as defined by the calculated influence that each device parameters specifying the sterilizer load has on each process step. In particular, the resulting sterilization sequence as described in the following, illustrates what takes place within a sterilizer.

111 represents the starting point for the air removal step shown in 110; the ramp 112 follows the decrease in pressure reaching a plateau 113 of a defined time duration at the reached vacuum pressure; after said time, the pressure is increased as shown by the ramp 114 above atmospheric pressure 100 and suddenly decreased 115 to a new value of vacuum pressure, which is maintained for a short duration of time. Upon completion of the air removal step 110, the system enters the sterilization step 120 and the pressure is increased 121 far above atmospheric pressure 100, with subsequent increase in temperature; the reached pressure 122 is maintained for 5 min, according to the table in Figure 1, during which the sterilization step 120 takes place; after sterilization 120, the pressure is again decreased as shown by the ramp 123 representing the venting phase, and the subsequent drying step 130 is entered further decreasing the pressure below atmospheric level, and maintaining the reached value for a set duration of time 131 according to the results of the table in Figure 1 to achieve the first stage of vacuum drying. Pulses of different duration composed of venting phase132, rapid pressure decrease 133, a vacuum drying plateau 134 and a further pulse 135 lead to completion of the drying step 136 and conclusion of the sterilization sequence.

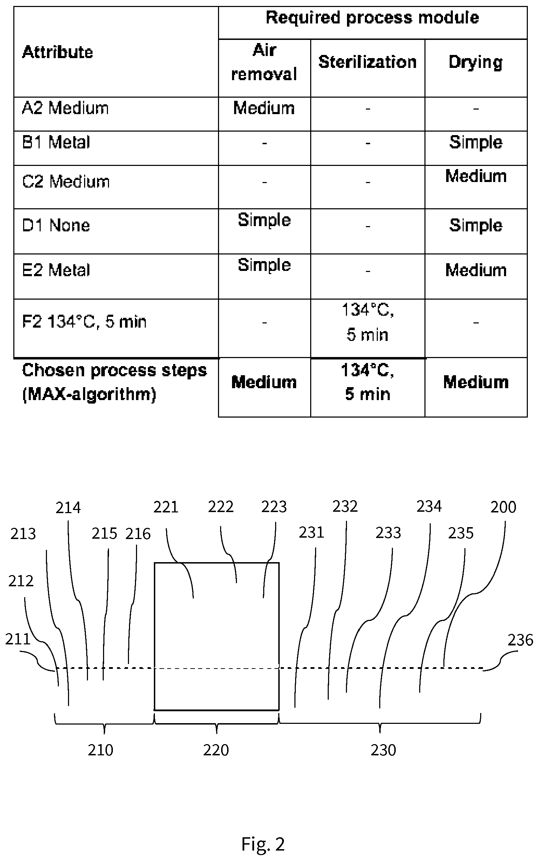

### Example 2: Full load with medium complex instruments as shown in Figure 2

The sterilizer is loaded fully with sets of instruments with simple and medium complex design, partially incorporating hollow parts (e.g. scissors, forceps, laparoscopic sheath, etc...). Upon specification of the sterilizer load the challenge for air removal is detected as medium.

The sets are located in a perforated inner tray contained within a rigid reusable sterilizing container. The challenge for drying is medium.

According to these attributes, the goods should be sterilized at 134° for 5 minutes.

The determination for the sterilization process sequence (table) and the customized sequence would look as shown in Figure 2.

In particular, Figure 2 is concerned with obtaining a customized sterilization sequence for a full load with medium complex instruments. The table in Figure 2 reports the device parameters as specified, deriving from a selection of categories and attributes within each category; the graph in Figure 2 shows a graphical representation of the sterilization sequence, as defined by the calculated influence that each device parameters specifying the sterilizer load has on each process step. In particular, the resulting sterilization sequence as described in the following, illustrates what takes place within a sterilizer.

211 represents the starting point for the air removal step shown in 210; the ramp 212 follows the decrease in pressure reaching a plateau 213 of a defined time duration at the reached vacuum pressure; after said time, the pressure is increased as shown by the ramp 214 above atmospheric pressure 200 and suddenly decreased 215 to a new value of vacuum pressure, suddenly slightly increased, after which the new value achieved is maintained for a short duration of time, before a new pulse 216 is performed with rapid venting up to atmospheric temperature and rapid vacuum to the same vacuum level as the previous pulse and sudden increase towards higher pressure leading to the next step of sterilization 220. The pressure is therefore increased 221 far above atmospheric pressure, with subsequent increase in temperature; the reached pressure 222 is maintained for 5 min, according to the table in Figure 2, during which the sterilization step 220 takes place; after sterilization 220, the pressure is again decreased as shown by the ramp 223 representing the venting phase, and the subsequent drying step 230 is entered further decreasing the pressure below atmospheric level 200, and maintaining the reached value for a set duration of time 231 according to the results of the table in Figure 2 to achieve the first stage of vacuum drying. Pulses of different duration composed of venting phase232, rapid pressure decrease 233, a vacuum drying plateau 230 and a further pulse 235 as the one described, lead to completion of the drying step 236 and conclusion of the sterilization sequence.

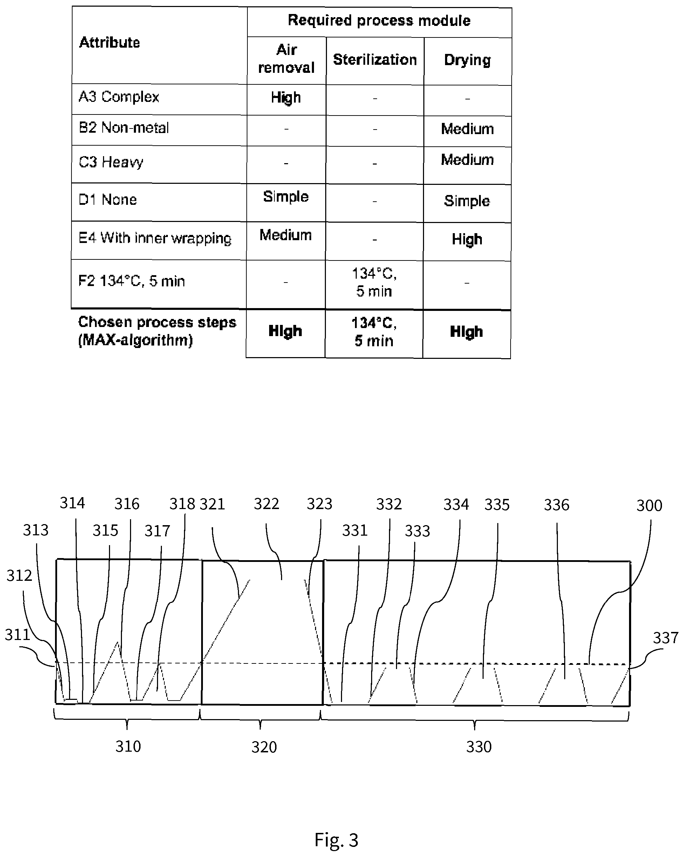

### Example 3: Full load with heavy and complex instruments as shown in Figure 3

The sterilizer is loaded fully with mixed sets of instruments, also containing complex instruments (e.g. drills, cannulated screw drivers, etc...). Upon specification of the sterilizer load the challenge for air removal is high.

The sets are heavy, the instruments are located in a perforated inner tray. The tray is wrapped and contained within a rigid reusable sterilizing container. Upon specification of the sterilizer load the challenge for drying is detected as high.

According to these attributes, the goods should be sterilized at 134° for 5 minutes.

The determination for the sterilization process sequence (table) and the customized sequence would look as shown in Figure 3.

In particular, Figure 3 is concerned with obtaining a customized sterilization sequence for a full load with heavy and complex instruments. The table in Figure 3 reports the device parameters as specified, deriving from a selection of categories and attributes within each category; the graph in Figure 3 shows a graphical representation of the sterilization sequence, as defined by the calculated influence that each device parameters specifying the sterilizer load has on each process step. In particular, the resulting sterilization sequence as described in the following, illustrates what takes place within a sterilizer.

311 represents the starting point for the air removal step shown in 310; the ramp 312 follows the decrease in pressure reaching a plateau 313 of a defined time duration at the reached vacuum pressure; after said time, the pressure is further decreased to a lower vacuum for a similar time duration 314, then increased as shown by the ramp 315 above atmospheric pressure 300 and suddenly decreased 316 to a new higher value of vacuum pressure, which is held for a defined time interval suddenly slightly increased, after which the new value achieved is maintained for a short duration of time, before a new pulse 317 is performed with rapid venting up to atmospheric temperature and rapid vacuum to the same vacuum level as the previous pulse and sudden increase towards higher pressure leading to the next step of sterilization 320. The pressure is therefore increased 321 far above atmospheric pressure 300, with subsequent increase in temperature; the reached pressure 322 is maintained for 5 min, according to the table in Figure 3, during which the sterilization step 320 takes place; after sterilization 320, the pressure is again decreased as shown by the ramp 323 representing the venting phase, and the subsequent drying step 330 is entered, further decreasing the pressure below atmospheric level, and maintaining the reached value for a set duration of time 331 according to the results of the table of Figure 3 to achieve the first stage of vacuum drying. Venting 332 follows to a higher pressure value, maintained for a defined duration 333, followed from venting 334 to the previous vacuum level maintained for the same duration and again venting leading to the repetition of the last cycle two more times, 335 and 336, leading to completion of the drying step 336 and conclusion of the sterilization sequence.

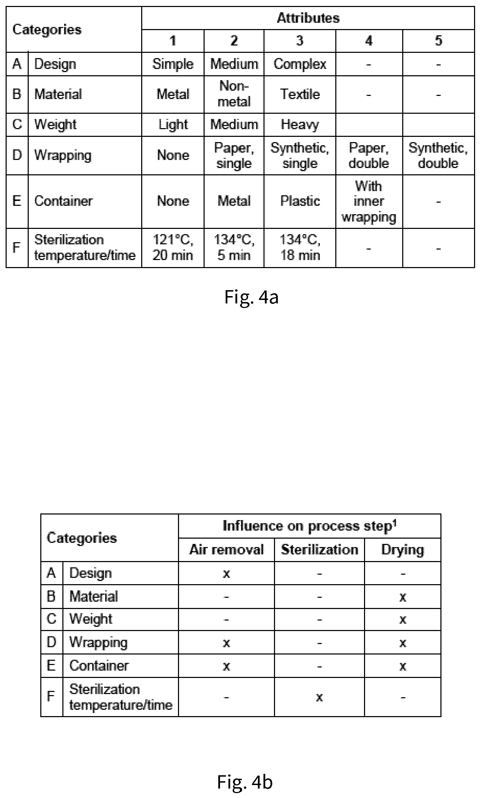

The table of Figure 4a represents a list of device parameters resulting from the specifications of categories and attributes within each category that best describe the device or sterilizer load. The table of Figure 4b represents the influence that each of the selected device parameters is calculated to have on a specific sterilization step. Based on these tables, a sterilization sequence is eventually determined that may include sterilization steps as shown in Examples 1 to 3 and Figures 1 to 3.

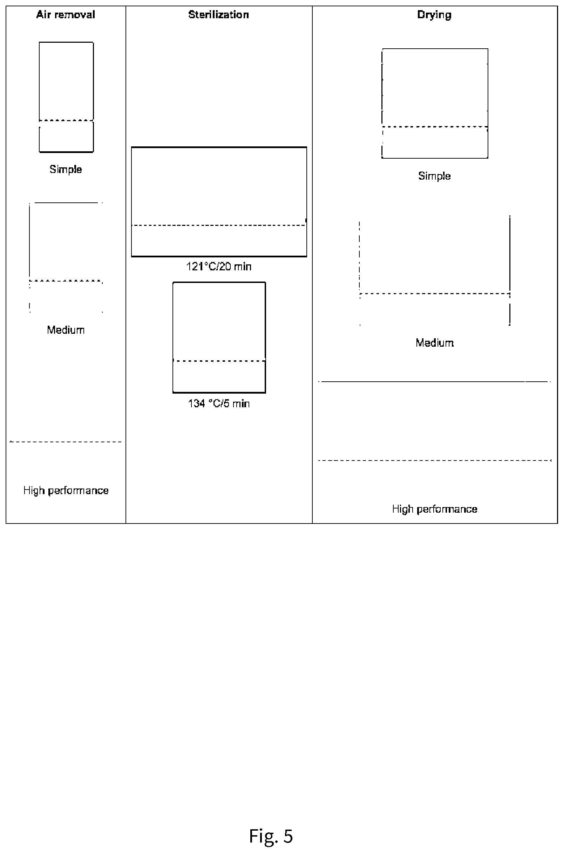

Figure 5 shows a visualization of what the different sterilization steps of air removal, sterilization and drying look like, including alternative forms obtained with different process parameters. Some of the sequences shown in this figure have been utilized in the explicative Examples 1 to 3 and shown in detail in Figure 1 to 3. The components of each step are illustrated and explained in Examples 1 to 3.

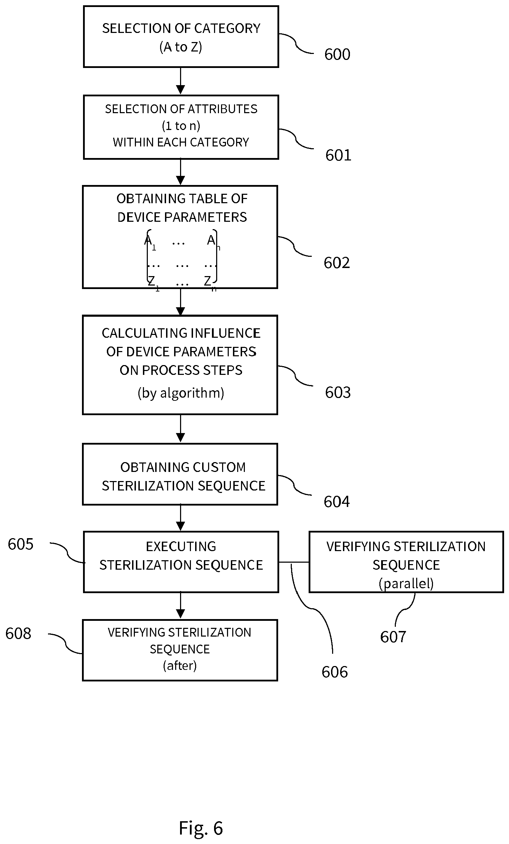

Figure 6 shows a flow chart illustrating the steps of the method according to the present invention through a block diagram. After the device or sterilizer load is loaded into the sterilizer, the selection of a category 600 form a list from A to Z of suitable categories is carried out, followed by a selection of the attributes 601 for each category, obtaining a table 602 listing a number of device parameters, as shown in Figure 4a. An algorithm, that in the Examples 1 to 3 is MAX-algorithm, is used to calculate the influence 603 that each device parameters from the table has on a specific step of the sterilization process. Once such influence has been calculated, for example as shown in Figure 4b, a custom sterilization sequence is obtained 604, which is then executed 605. A further verification step 607 or 608 is comprised, that can be run either parallel 606 to the sterilization sequence 607, or that can be run after the completion of the sterilization sequence 608, without necessitating of the device to be present in the sterilizer.

### List of elements

- 100: Atmospheric pressure
- 110: air removal step
- 111: starting point
- 112: pressure plunge below ca. 1013 hPa
- 113: vacuum plateau
- 114: venting/rising pressure
- 115: lowering pressure

- 120: sterilization step
- 121: pressurizing
- 122: high pressure plateau
- 123: venting

- 130: drying step
- 131: vacuum plateau
- 132: venting
- 133: rapid decrease in pressure
- 134: vacuum plateau
- 135: further repetition of 132 and 133 followed by shorter vacuum plateau and 132
- 136: end of process

- 200: Atmospheric pressure
- 210: air removal step
- 211: process starting point
- 212: pressure plunge below 1013 hPa
- 213: vacuum plateau
- 214: venting/rising pressure
- 215: rapid pressure plunge
- 216: further pulse

- 220: sterilization step
- 221: pressurizing
- 222: high pressure plateau
- 223: venting

- 230: drying step
- 231: vacuum plateau
- 232: venting
- 233: rapid pressure plunge
- 234: vacuum plateau
- 235: further repetition of 232, 233, 234 and 232
- 236: end of process

- 300: Atmospheric pressure
- 310: air removal step
- 311: process starting point
- 312: pressure plunge below 1013 hPa
- 313: vacuum plateau
- 314: further pressure plunge and vacuum plateau
- 315: venting to above 1013 hPa
- 316: rapid pressure plunge below 1013 hPa
- 317: vacuum plateau
- 318: further pulse consisting of rapid venting to 1013 hPa followed by rapid pressure plunge and further vacuum plateau

- 320: sterilization step
- 321: pressurizing
- 322: high pressure plateau
- 323: venting

- 330: drying step
- 331: vacuum plateau
- 332: venting
- 333: vacuum plateau
- 334: pressure plunge
- 335: further repetition of 331, 332, 333 and 334
- 336: further repetition of 335 followed by shorter vacuum plateau and venting
- 337: end of process

- 600: step of selection of category for device parameters
- 601: step of selection of attributes within a category of device parameters
- 602: step of obtaining table of device parameters
- 603: step of calculating the influence of device parameters on the sterilization process steps
- 604: step of obtaining a custom sterilization sequence
- 605: step of executing the custom sterilization sequence
- 606: connector indicating parallel execution
- 607: verification step run in parallel to sterilization sequence
- 608: verification step after completion of sterilization sequence

## Claims

1. A method of providing a customizable sterilization process for a sterilizer load or device, comprising the steps of:
a. Specifying the device by its characterizing parameters from a list of device parameters, wherein each device parameter possesses an identification code/tag;
b. Providing a list for different process steps including air removal, sterilization and drying, together with individual process settings for each individual process step;
c. Using the identification code/tag for selecting a sterilization sequence including the process steps and the individual process settings from the list;
d. Execution of the sterilization sequence.

2. The method according to claim 1, **characterized in that** the list of device parameters comprises at least one of device parameters chosen from a list of categories of device parameters, wherein the categories range e.g. from A to Z.

3. The method according to claim 1 or 2, **characterized in that** each category of device parameters comprises e.g. from 1 to *n* device parameters, wherein the number of device parameters is defined by the number of attributes of a given category.

4. The method according to claim 1 to 3, **characterized in that** the identification code/tag corresponds to the combination of a category identifier e.g. from A to Z and the number of device parameters per category e. g. from 1 to *n.*

5. The method according to claim 1 to 4, **characterized in that** the list of categories of device parameters includes at least one of e.g. design, including structural parameters such as e.g. the presence of cavities and/or recesses and/or protrusions; weight; size; material or materials of the device; wrapping of the device; container of the device; sterilization temperature and sterilization time of the device; and/or wherein the list of attributes of device parameters for each category includes at least one of a list of specific attributes of a category, wherein e.g. the category design includes at least one of the attributes such as easy, medium, complex.

6. The method according to claims 1 to 5, **characterized in that** each device parameter has an associated influence on a given process step, wherein each device parameter is associated with a suitable process parameter for any given sterilization step, and wherein a sterilization sequence is therefore built by cross-correlating the device parameters with the corresponding sterilization steps.

7. The method according to claims 1 to 6, **characterized in that** the specification step comprises at least one of the steps of (i) selecting a category from a list of categories of device parameters; (ii) selecting an attribute for the device parameter in the given category; (iii) repeating (i) and (ii) for each relevant category.

8. The method according to claims 1 to 7, **characterized in that** it further includes one or more of the following steps of:
a. providing an automatic selection of a batch monitoring and indicator system suitable for a given sterilization sequence, wherein the suitable batch monitoring and indicator system is identified by a code/tag.
b. monitoring and/or verification of the results of the executed sterilization sequence, wherein the monitoring and/or verification step is continuous, batch or on demand, and/or wherein the verification step takes place without any device, and preferably with a daily frequency, and/or wherein the verification step taking place without any device or sterilizer load is carried out by placing a commercial analogue indicator e.g. based on colour and/or turbidity change, such as an indicator paper, indicator test strips, indicator tape, indicator solution, spore ampoules, chemical indicator, steam chemical process indicator, biological indicator, self-contained biological indicator, bowie and dick test, or digital monitors/indicators, and/or any combination thereof.

9. The method according to claims 1 to 8, **characterized in that** the verification of sterilization by batch monitoring is carried out in parallel to a sterilization sequence.

10. The method according to claim 1 to 9, **characterized in that** any of the steps of the sterilization sequence of air removal, sterilization, and drying may be performed more than once, each step also with different parameters.

11. The method according to claims 1 to 10, **characterized in that** any step of the sterilization sequence has one or more alternative forms, wherein an alternative form includes a set of parameters, such as, e.g., method, pressure, duration, temperature, gradients, pulses.

12. The method according to claims 1 to 11, **characterized in that** the step of air removal is carried out according to one or more of suitable air removal methods e.g. pre-vacuum air removal, fractionated pre-vacuum air removal, gravitation method, and/or using steam pulses, such as e.g. atmospheric pulses, trans-atmospheric pulses, overpressure pulses, and/or at a vacuum pressure in the range from 10 mbar to 200 mbar, and in particular from 20 mbar to 150 mbar, and preferably from 30 mbar to 100 mbar, and/or according to rising and/or falling gradients.

13. The method according to claims 1 to 12, **characterized in that** the step of sterilization is carried out at temperatures in the range from 100°C to 160°C, alternatively from 110°C to 140°C, in particular from 120°C to 135°C and preferably at 121° C or 134° C; and/or has durations or holding times in the range from 1 min to 30 min, and alternatively from 2 min to 25 min, in particular from 3.5 min to 20 min, and preferably from 5 min to 10 min, according to the sterilization temperature.

14. The method according to claims 1 to 13, **characterized in that** the step of drying is carried out according to one or more of suitable drying methods e.g. vacuum drying, fractionated drying with air admission, fractionated drying with steam admission; and/or using vacuum drying in combination with air or steam pulses, such as e.g. atmospheric pulses, trans-atmospheric pulses, overpressure pulses, air pulses, steam pulses, wherein the number of pulses is in the range from above 0 to 10, and in particular from 2 to 8, and preferably from 4 to 6, and the duration of said pulses is in the range from above 0 min to 10 min, and in particular from 1 min to 8 min, and preferably from 2 min to 4 min, and from above 4 min to 6 min, and wherein the air temperature is in the range from 5°C to 160°C, and in particular from 10°C to 140°C, and preferably from 20°C to 120°C, in particular in combination with improved convection, e.g. by ventilation at a vacuum pressure in the range from 10 mbar to 200 mbar, and in particular from 20 mbar to 150 mbar, and preferably from 30 mbar to 100 mbar; and/or wherein the step of drying has durations in the range from 1 min to 60 min, and in particular from 5 min to 45 min, and preferably from 10 min to 30 min.

15. The use of the method according to any of the preceding claims for the sterilization of devices in any private and public industries where a standard hygiene requirement has to be met, such as e.g. hospitals, clinics, dentistry and orthodontics laboratories, the semiconductor manufacturing industry, the food handling, producing and manufacturing industry, the space technology manufacturing industry, the pharmaceutical industry, clean room environments, life science laboratories, animal facilities, beauty and cosmetic treatments salon, tattoo parlours.
